# EUROPEAN PATENT SPECIFICATION

(11) **EP 0 983 035 B1**
(45) Date of publication and mention of the grant of the patent: **19.05.2004**
(21) Application number: 99913436.4
(22) Date of filing: 19.03.1999
(51) Int. Cl.: A61F 2/30

(54) **CENTRALISER**
ZENTRIERELEMENT
ELEMENT DE CENTRAGE

(30) Priority: 20.03.1998 GB 9806053; 24.03.1998 GB 9806282; 07.10.1998 GB 9821858
(43) Date of publication of application: 08.03.2000
(73) Proprietor: Johnson & Johnson Medical Limited, Ascot, Berkshire RG12 2AT (GB)
(72) Inventor: GRIFFITHS, Brian, Lymington, Hampshire (GB); REVIE, Ian, New Milton, Hampshire (GB)
(74) Representative: Fisher, Adrian John
(86) International application number: PCT/GB1999/000884
(87) International publication number: WO 1999/048443

(56) References cited:
- EP-A- 0 363 151
- EP-A- 0 427 444
- WO-A-91/03992
- WO-A-95/13757
- WO-A-97/04723

## Description

This invention relates to a centraliser for a prosthetic implant, in particular a prosthetic femoral stem for implantation in a patient's femur for hip replacement surgery.

Centralisers or spacers are provided for fitting to the distal end of a femoral hip replacement stem in order to keep the implant stem away from the internal surface of the cavity of the bone in which the stem is being inserted. In the case of stems which are cemented in the bone cavity, there is a space between the stem and the internal surface of the cavity of the bone in which bone cement is placed. Controlling the position of the stem within the surrounding bone cement mantle is vital to the longterm survivability of the replacement joint. Cement can be deposited in the bone cavity and the stem with attached centraliser can be inserted. It is important to try to obtain an even and intact cement mantle around the stem.

Known centralisers are in the form of a cap which fits over the distal end of the stem such as shown in EP-A-427 444 or are in the form of centralisers which are fixed inside the drilled end of the stem. Centralisers are also known, for example as described in US patent no. 5,658,351 of ring form which can have a tapered inner surface corresponding to the tapered surface of the distal end of a femoral stem on which the centraliser is located.

Problems can arise in locating a centraliser on the tip of a tapering stem. The location can be achieved by close fitting of the centraliser by shaping the inside of the centraliser to the stem to ensure suitable positioning. The problem associated with shaping the internal surface of the centraliser is that the fit to the femoral stem is unidirectional and incorrect fitting could cause fracture of the centraliser due to the elevation of hoop stresses on the centraliser caused by point or ring loading. The centraliser could be marked to indicate the correct orientation; however, orientation marking is very difficult due to the size of the centraliser.

An object of the present invention is to ensure correct positioning of a centraliser with no orientation problems.

According to a first aspect of the present invention, there is provided a centraliser for use on a prosthetic femoral stem to be inserted in a bone cavity, the centraliser comprising a hollow body having an internal surface and an external surface and spacer means projecting from the body, wherein the internal surface is of convex curvature.

Preferably, the radius of curvature of the internal surface is in the range of 20-100mm. Most preferably, the radius of curvature of the internal surface is in the range of 30-50mm, optimally 40mm.

Preferably, the spacer means has an outer edge of convex curvature for contacting an internal surface of the bone cavity. The radius of curvature of the outer edge of the spacer means may be in the range of 10-100mm. Preferably, the radius of curvature of the outer edge of the spacer means is in the range of 10-40mm.

The spacer means may be in the form of a plurality of fins extending from the external surface of the hollow body. The hollow body may be of ring form with two open ends joined by the internal and external surfaces.

Preferably, a mid-portion of the hollow body is of increased thickness.

Preferably, the external surface of the hollow body of the centraliser is also of convex curvature. The radius of curvature of the external surface may be in the range of 10-100mm. Preferably, the radius of curvature of the external surface is in the range of 10-40mm.

Preferably, the radius of curvature of the outer edge of the spacer means and radius of curvature of the external surface of the hollow body are approximately equal.

The hollow body of the centraliser may have a generally rectangular or oval cross-section.

In the case of a rectangular cross-section, the internal corners of the generally rectangular hollow body have indented reliefs.

Preferably, the fins extend radially from the outer surface of the hollow body and longitudinally in the direction of a femoral stem on which the centraliser is disposed. In the case where four fins are provided, one fin corresponds to each of the anterior, posterior, medial and lateral surfaces of the femoral stem.

An embodiment of a centraliser in accordance with the present invention will now be described, by means of example only, with reference to the accompanying drawings, in which:
Figure 1 is a cross-sectional view of the centraliser in position on a distal end of a femoral stem prosthesis with variations in stem size shown in broken lines;
Figure 2 is a cross-section through line A-A of the centraliser and femoral stem of Figure 1;
Figure 3 is a cross-section of a prior art centraliser located on a distal end of a femoral stem illustrating the instability of the prior art centraliser;
Figure 4 is a cross-section of the centraliser of Figure 1 disposed at an angle on the femoral stem; and
Figure 5, which does not form part of the invention, is a diagrammatic representation of a cross-section of the centraliser of Figure 1 disposed on a femoral stem showing the relationship between the taper angle of the femoral stem, the radius of curvature R of the internal surface of the centraliser and the dimensions L, T of the centraliser.

Referring to the drawings, a centraliser 1 is provided for use with a prosthetic femoral stem 2 for implantation in a patient's femur for hip replacement surgery. The centraliser 1 is located on a distal end 4 of the femoral stem 2.

In the described embodiment, the centraliser 1 is used with a tapered femoral stem 2 with anterior 6, posterior 8, medial 10 and lateral 12 surfaces which are generally flat resulting in a tapered rectangular form of cross-section of the stem 2 with the cross-sectional area decreasing towards the distal end 4 of the femoral stem 2. The centraliser of the present invention can be used with other forms of femoral stem and is not restricted to use with the tapered femoral stem 2 of the described embodiment.

Tapered femoral stems 2 can have polished surfaces which co-operate advantageously with the cement mantle resulting in reduced fractures in the cement.

The centraliser 1 has an elongated ring body 14 with a top edge 16 defining a first open end of the elongated ring body 14 and a bottom edge 18 defining the second open end. An internal surface 22 of the ring body 14 extends between the top edge 16 and the bottom edge 18. The internal surface 22 is of convex curvature between the top edge 16 and the bottom edge 18.

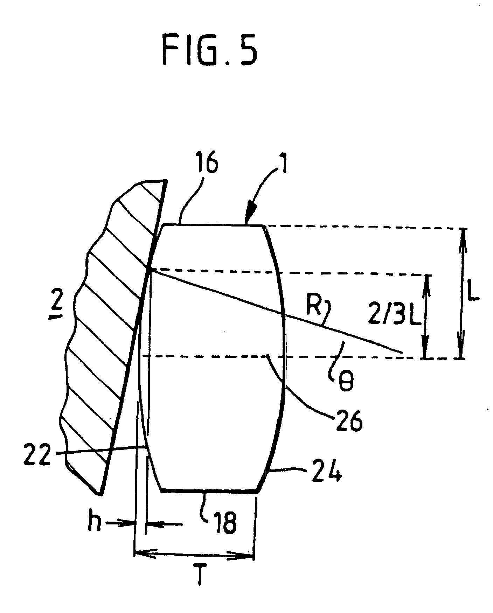

Figure 5, which does not form part of the invention, illustrates the optimum relationship between the internal radius of curvature R and dimensions of the centraliser 1 and the taper angle θ of the femoral stem 2.

Taper contact between the femoral stem 2 and the centraliser 1 is assumed to be at a maximum length of 2/3L to ensure the material is of adequate thickness for stress reduction, where L is half the height of the centraliser 1. For centreline contact between the femoral stem 2 and the centraliser 1, θ=0 and R⇒∞, where R is the radius of curvature of the internal surface 22 of the centraliser 1.

At 2/3L contact the following equations apply:
The taper angle/radius relationship is : Sinθ=2L/3R
   h=R-√(R²-(2/3L)²)
   2/3L²=h(2R-h)

Wherein h is the distance between the surface of the centraliser at the centre-line and at 2/3L.

An optimal radius of curvature R of the internal surface 22 of the centraliser 1 for use with the tapered femoral stem 2 of the described embodiment is approximately R=40mm, or in the range of R=30-50mm. Although a radius of curvature of the range R=20-100mm would also work. In the preferred embodiment in which R=40mm, L=5mm, T=2mm for use with a taper angle of θ=3°.

The ring body 14 has an external surface 24 which extends between the top edge 16 and the bottom edge 18. The external surface 24 has a convex curvature. The radius of the external surface 24 of the centraliser 1 is as small as possible (i.e. close to L). In the described embodiment the radius of curvature of the external surface 24 is approximately 40mm or in the range of 10-40mm, although a radius of curvature in the range of 10-100mm would also be acceptable.

A mid-portion 26 of the ring body 14 has a greater thickness than the top edge 16 and the bottom edge 18.

In the described embodiment. the centraliser I has a generally rectangular cross-section formed of four sides 30 as shown in Figure 2. The centraliser 1 has comer reliefs 28 at the internal comers of the rectangular cross-section for increased flexibility. The four sides 30 of the centraliser 1 correspond to and contact the anterior 6, posterior 8, medial 10 and lateral 12 surfaces of the femoral stem 2. Each of the sides 30 of the centraliser 1 has a fin 20 which extends radially from the external surface 24 of the centraliser 1. The fins 20 are located centrally on each side 30 of the centraliser 1 and extend longitudinally between the top edge 16 and the bottom edge 18 of the centraliser 1. A centraliser 1 in accordance with the present invention may have any suitable shape of cross-section to correspond to the type of femoral stem 2 with which it is to be used.

The fins 20 have outside edges 32 which contact the internal surfaces 34 of the cavity in the patient's bone in which the femoral stem 2 and the centraliser 1 are inserted. The edges 32 of the fins 20 have convex curvature. The convex curvature of the fins 20 has a radius of curvature of approximately 40mm or in the range of 10-40mm although a radius of curvature in the range of 10-100mm would be acceptable. The radius of curvature of the edges 32 of the fins should correspond to the radius of curvature of the external surface 24 of the ring body 14.

In use, the centraliser 1 is disposed on the distal end 4 of the femoral stem 2 before insertion of the femoral stem 2 into a cavity in a patient's femur.

Due to the convex curvature of the internal surface 22 of the centraliser 1, the centraliser 1 can be easily orientated on the distal end 4 of the femoral stem 2. The contact between the internal surface 22 of the centraliser 1 and the surface of the femoral stem 2 is a point or ring contact. The contact is in the mid-portion 26 of the centraliser 1 where the material thickness reduces the effect of the hoop loading to a level where it is no longer of concern.

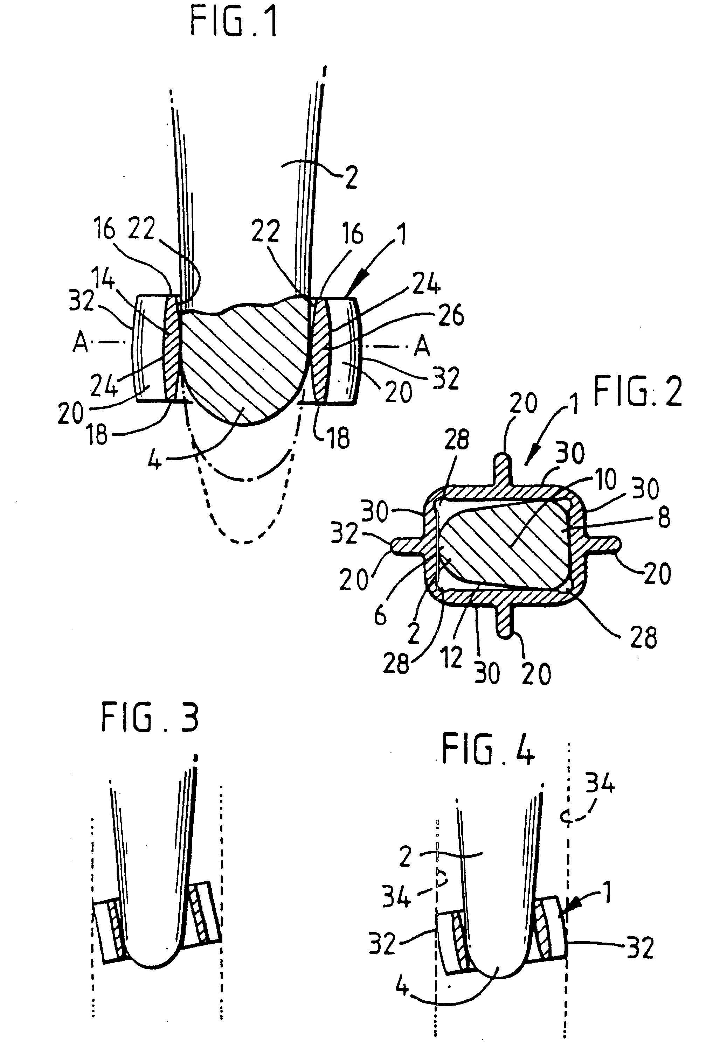

The convex curvature of the internal surface 22 of the centraliser allows the centraliser 1 to be placed unevenly on the femoral stem 2 whilst maintaining the correct cement mantle as shown in Figure 4. This was not possible with centralisers known from the prior art which did not have internal surfaces with a convex curvature as shown in Figure 3.

The convex curvature of the edges 32 of the fins 20 allows the centraliser 1 to be held securely within the bone cavity even when it is misaligned with the femoral stem 2. If the relationship between the curvature of the edges 32 of the fins 20 and the angle of the internal surface of the bone cavity is adapted to correspond to the relationship between the curvature of the internal surface of the centraliser 1 and the angle of the tapered femoral stem, the centraliser 1 can be positioned in a stable manner even if it is misaligned with the femoral stem.

The external surface 24 can have a convex curvature to help direct hoop stresses to the thickest portion of the ring body 14. Corresponding the curvature of the external surface 24 to the curvature of the edges of the fins 20 has the benefit removing any interruption of the cement mantle by the ring body 14 in the event that the centraliser 1 is misaligned as shown in Figure 4.

The centraliser 1 has the added advantage that at can be used on a number of different sizes of femoral stems 2 as shown in Figure 1.

The edges 32 of the fins 20 contact the internal surfaces 34 of the prepared bone cavity thereby positioning the femoral stem 2 centrally in the cavity.

Cement can be introduced into the cavity in which the femoral stem 2 and centraliser 1 have been placed. The fins 20 provide minimal interruption to the cement flow past the fins 20.

It will be appreciated that the centraliser 1 can have a circular, or other shaped, cross-section and any number of fins 20 greater than two.

Modifications and improvements can be made to the foregoing without departing from the scope of the present invention as defined in the claims.

## Claims

1. A centraliser for use on a prosthetic femoral stem to be inserted in a bone cavity, the centraliser comprising a hollow body (14) having an internal surface (22) and an external surface (24) and spacer (20) means projecting from the body, **characterised in that** the internal surface (22) is of convex curvature.

2. A centraliser as claimed in claim 1, wherein the radius of curvature of the internal surface is in the range of 20-100mm.

3. A centraliser as claimed in claim 2, wherein the radius of curvature of the internal surface is in the range of 30-50mm.

4. A centraliser as claimed in any of the preceding claims, wherein the spacer means (20) has an outer edge (32) of convex curvature for contacting an internal surface of the bone cavity.

5. A centraliser as claimed in claim 4, wherein the radius of curvature of the outer edge of the spacer means is in the range of 10-100mm.

6. A centraliser as claimed in claim 5, wherein the radius of curvature of the outer edge of the spacer means is in the range of 10-40mm.

7. A centraliser as claimed in any of the preceding claims, wherein the spacer means is in the form of a plurality of fins extending from the external surface of the hollow body.

8. A centraliser as claimed in any of the preceding claims, wherein the hollow body is of ring form with two open ends joined by the internal and external surfaces.

9. A centraliser as claimed in any of the preceding claims, wherein a mid-portion of the hollow body is of increased thickness.

10. A centraliser as claimed in any of the preceding claims, wherein the external surface (24) of the hollow body of the centraliser is of convex curvature.

11. A centraliser as claimed in claim 10, wherein the radius of curvature of the external surface is in the range of 10-100mm.

12. A centraliser as claimed in claim 11, wherein the radius of curvature of the external surface is in the range of 10-40mm.

13. A centraliser as claimed in any of claims 10 to 12, wherein the radius of curvature of the outer edge (32) of the spacer means (20) and radius of curvature of the external surface (24) of the hollow body are equal.

14. A centraliser as claimed in any of the preceding claims, wherein the hollow body has a generally rectangular or oval cross-section.

15. A centraliser as claimed in claim 14, wherein the internal comers of the generally rectangular hollow body have indented reliefs (28).

16. A centraliser as claimed in any of claims 7 to 15, wherein the fins extend radially from the outer surface of the hollow body and longitudinally in the direction of a femoral stem on which the centraliser is disposed.

17. A centraliser as claimed in claim 16, wherein four fins are provided, one fin corresponding to each of the anterior, posterior, medial and lateral surfaces of the femoral stem.

## Patentansprüche

1. Zentrierer zur Nutzung in einem in einen Knochenhohlraum einzuführenden Prothesen-Oberschenkelstab mit einem Hohlkörper (14), der eine innere Oberfläche (22) und eine äußere Oberfläche (24) aufweist, und Abstandsmitteln (20), die von dem Körper abstehen, **dadurch gekennzeichnet, daß** die innere Oberfläche (22) eine konvexe Krümmung aufweist.

2. Zentrierer nach Anspruch 1, **dadurch gekennzeichnet, daß** der Radius der Krümmung der inneren Oberfläche im Bereich von 20 - 100 mm liegt.

3. Zentrierer nach Anspruch 2, **dadurch gekennzeichnet, daß** der Radius der Krümmung der inneren Oberfläche im Bereich von 30 - 50 mm liegt.

4. Zentrierer nach einem der vorangehenden Ansprüche, **dadurch gekennzeichnet, daß** die Abstandsmittel (20) einen äußeren Rand (32) mit einer konvexen Krümmung zum Berühren einer inneren Oberfläche des Knochenhohlraums aufweisen.

5. Zentrierer nach Anspruch 4, **dadurch gekennzeichnet, daß** der Radius der Krümmung des äußeren Rands der Abstandsmittel im Bereich von 10 - 100 mm liegt.

6. Zentrierer nach Anspruch 5, **dadurch gekennzeichnet, daß** der Radius der Krümmung des äußeren Rands der Abstandsmittel im Bereich von 10 - 40 mm liegt.

7. Zentrierer nach einem der vorangehenden Ansprüche, **dadurch gekennzeichnet, daß** die Abstandsmittel mehrere Rippen sind, die sich von der äußeren Oberfläche des Hohlkörpers erstrecken.

8. Zentrierer nach einem der vorangehenden Ansprüche, **dadurch gekennzeichnet, daß** der Hohlkörper eine Ringform mit zwei offenen Enden aufweist, die durch die innere und die äußere Oberfläche verbunden sind.

9. Zentrierer nach einem der vorangehenden Ansprüche, **dadurch gekennzeichnet, daß** ein Mittelabschnitt des Hohlkörpers eine größere Dicke aufweist.

10. Zentrierer nach einem der vorangehenden Ansprüche, **dadurch gekennzeichnet, daß** die äußere Oberfläche (24) des Hohlkörpers des Zentrierer eine konvexe Krümmung aufweist.

11. Zentrierer nach Anspruch 10, **dadurch gekennzeichnet, daß** der Radius der Krümmung der äußeren Oberfläche im Bereich von 10 - 100 mm liegt.

12. Zentrierer nach Anspruch 11, **dadurch gekennzeichnet, daß** der Radius der Krümmung der äußeren Oberfläche im Bereich von 10 - 40 mm liegt.

13. Zentrierer nach einem der Ansprüche 10 - 12, **dadurch gekennzeichnet, daß** der Radius der Krümmung des äußeren Rands (32) der Abstandsmittel (20) und der Radius der Krümmung der äußeren Oberfläche (24) des Hohlkörpers gleich sind.

14. Zentrierer nach einem der vorangehenden Ansprüche, **dadurch gekennzeichnet, daß** der Hohlkörper einen im wesentlichen rechteckigen oder ovalen Querschnitt aufweist.

15. Zentrierer nach Anspruch 14, **dadurch gekennzeichnet, daß** die inneren Ecken des im wesentlichen rechteckigen Hohlkörpers gezahnte Aussparungen (28) aufweisen.

16. Zentrierer nach einem der Ansprüche 7 - 15, **dadurch gekennzeichnet, daß** die Rippen sich von der äußeren Oberfläche des Hohlkörpers radial und in Längsrichtung eines Oberschenkelstabs erstrecken, auf dem der Zentrierer angeordnet ist.

17. Zentrierer nach Anspruch 16, **dadurch gekennzeichnet, daß** vier Rippen vorgesehen sind, wobei jeweils eine Rippe der vorderen, der hinteren, der mittleren und der seitlichen Oberfläche des Oberschenkelstabs entspricht.

## Revendications

1. Elément de centrage pour utilisation sur une diaphyse fémorale prothétique à insérer dans une cavité osseuse, l'élément de centrage comprenant un corps creux (14) ayant une surface interne (22) et une surface externe (24) et un moyen d'espacement (20) faisant saillie depuis le corps, **caractérisé en ce que** la surface interne (22) a une courbure convexe.

2. Elément de centrage selon la revendication 1, dans lequel le rayon de courbure de la surface interne est dans la plage de 20 à 100 mm.

3. Elément de centrage selon la revendication 2, dans lequel le rayon de courbure de la surface interne est dans la plage de 30 à 50 mm.

4. Elément de centrage selon une quelconque des revendications précédentes, dans lequel le moyen d'espacement (20) a un bord extérieur (32) de courbure convexe pour faire contact avec une surface interne de la cavité osseuse.

5. Elément de centrage selon la revendication 4, dans lequel le rayon de courbure du bord extérieur du moyen d'espacement est dans la plage de 10 à 100 mm.

6. Elément de centrage selon la revendication 5, dans lequel le rayon de courbure du bord extérieur du moyen d'espacement est dans la plage de 10 à 40 mm.

7. Elément de centrage selon une quelconque des revendications précédentes, dans lequel le moyen d'espacement est en forme d'une pluralité d'ailettes s'étendant depuis la surface externe du corps creux.

8. Elément de centrage selon une quelconque des revendications précédentes, dans lequel le corps creux est de forme annulaire avec deux extrémités ouvertes, reliées aux surfaces interne et externe.

9. Elément de centrage selon une quelconque des revendications précédentes, dans lequel une portion de milieu du corps creux a une épaisseur accrue.

10. Elément de centrage selon une quelconque des revendications précédentes, dans lequel la surface externe (24) du corps creux de l'élément de centrage a une courbure convexe.

11. Elément de centrage selon la revendication 10, dans lequel le rayon de courbure de la surface externe est dans la plage de 10 à 100 mm.

12. Elément de centrage selon la revendication 11, dans lequel le rayon de courbure de la surface externe est dans la plage de 10 à 40 mm.

13. Elément de centrage selon une quelconque des revendications 10 à 12, dans lequel le rayon de courbure du bord extérieur (32) du moyen d'espacement (20) et le rayon de courbure de la surface externe (24) du corps creux sont égaux.

14. Elément de centrage selon une quelconque des revendications précédentes, dans lequel le corps creux a une section transversale généralement rectangulaire ou ovale.

15. Elément de centrage selon la revendication 14, dans lequel les coins internes du corps creux généralement rectangulaire a des reliefs dentelés (28).

16. Elément de centrage selon une quelconque des revendications 7 à 15, dans lequel les ailettes s'étendent radialement depuis la surface extérieure du corps creux et longitudinalement dans la direction d'une diaphyse fémorale sur laquelle l'élément de centrage est disposée.

17. Elément de centrage selon la revendication 16, dans lequel quatre ailettes sont fournies, une ailette chacune correspondant à chacune des surfaces antérieure, postérieure, médiale et latérale de la diaphyse fémorale.
